(19) Europäisches Patentamt
European Patent Office
Office européen des brevets

(11) **EP 1 656 883 A1**

(12) **DEMANDE DE BREVET EUROPEEN**

(43) Date de publication:
**17.05.2006 Bulletin 2006/20**

(51) Int Cl.:
*A61B 5/0488* (2006.01)   *A61B 5/04* (2006.01)

(21) Numéro de dépôt: **04447248.8**

(22) Date de dépôt: **10.11.2004**

(84) Etats contractants désignés:
**AT BE BG CH CY CZ DE DK EE ES FI FR GB GR HU IE IS IT LI LU MC NL PL PT RO SE SI SK TR**
Etats d'extension désignés:
**AL HR LT LV MK YU**

(71) Demandeur: **UNIVERSITE LIBRE DE BRUXELLES**
**1050 Bruxelles (BE)**

(72) Inventeurs:
• **Cantraine, Francis**
**1180 Bruxelles (BE)**

• **Mathys, Pierre**
**1440 Braine Le Chateau (BE)**
• **De Bel, Maxime**
**1410 Waterloo (BE)**

(74) Mandataire: **Van Malderen, Joelle et al**
**pronovem - Office Van Malderen**
**Avenue Josse Goffin 158**
**1082 Bruxelles (BE)**

(54) **Appareil de mesure portable d'un signal EMG**

(57)      La présente invention se rapporte à un appareil portable et autonome pour la mesure et la transmission à distance de signaux électromyographiques (EMG), comprenant au moins un stimulateur électrique (1), une paire d'électrodes d'acquisition du signal EMG (21, 31), une chaîne d'acquisition (2) pilotée par un microcontrôleur (3, 39) et reliée, via une interface standardisée (5), à un ordinateur (4) comprenant des moyens de mémorisation (9) et d'affichage des signaux EMG acquis ainsi qu'un programme exécutable pour réaliser l'interface avec l'utilisateur (6) et exploiter les données mémorisées, caractérisé en ce que la chaîne d'acquisition (2) comporte en outre des moyens d'élimination ou d'atténuation d'un artefact de stimulation présent dans le signal EMG, coopérant avec des moyens d'ajustement automatique du gain d'amplification du signal EMG (23, 24, 311, 38), via le microcontrôleur, de manière à ce que le signal EMG couvre la plus grande partie possible de la plage de tension d'entrée du CAN (26, 310), donc avec conservation de la résolution, lorsque l'amplitude du signal EMG diminue.

FIG.1

**Description**

**Objet de l'invention**

La présente invention se rapporte à un nouvel appareil de mesure d'électromyogrammes.

Lorsqu'un muscle est en activité, il est possible de recueillir un signal (bio)électrique de faible amplitude en plaçant des électrodes sur celui-ci. L'ensemble de ces signaux, se présentant sous forme de potentiels électriques, est appelé *électromyogramme* (EMG).

Le but d'une analyse EMG est d'obtenir de l'information sur l'état et le fonctionnement des muscles par la quantification de l'activité électromusculaire. Cette mesure est effectuée au moyen d'électrodes appliquées sur ou sous la peau. Un signal est détecté, traduisant l'activité du muscle sous-jacent.

**Arrière-plan technologique et état de la technique**

On connaît l'électro-stimulation qui consiste à exciter un nerf moteur périphérique à l'aide d'impulsions électriques pour provoquer, de manière externe, donc sans l'intermédiaire du cerveau, la réaction du muscle qui lui est associée.

Il convient donc de distinguer les signaux EMG, résultant d'une stimulation électrique, des signaux EMG spontanés, résultant d'un mouvement volontaire du muscle.

Lors d'une anesthésie générale, différentes drogues sont injectées au patient et ont pour but :

- d'assurer l'amnésie et le sommeil par l'inconscience ;
- d'insensibiliser à la douleur par l'analgésie et
- de permettre le relâchement musculaire.

Le curare diminuant le nombre de fibres musculaires actives, on recourt aux EMG pour évaluer le taux de relâchement musculaire.

Cette évaluation du relâchement musculaire est confrontée à un certain nombre de difficultés de mesure :

- une diminution de l'amplitude des EMG au cours de la curarisation ;
- un environnement bruité, notamment par la pollution électromagnétique ;
- l'exigence que la phase d'initialisation, c'est-à-dire le temps de pose des électrodes et le calibrage de l'appareil, soit relativement courte.

Le brevet US-A-4 291 705 décrit un appareil permettant de déterminer le degré de paralysie neuromusculaire, en effectuant une stimulation "supra-maximale" (voir ci-après, § [0157] et suivants) d'un nerf moteur périphérique. Cet appareil intègre le signal EMG redressé et affiche le résultat. L'état de paralysie est indiqué en mesurant le rapport de la surface de l'EMG curarisé sur la surface de l'EMG de référence, c'est-à-dire celui obtenu avant les premières injections de curare.

Le système décrit est entièrement analogique et fournit un signal de sortie proportionnel au rapport des surfaces des signaux EMG redressés. Ce signal analogique est dirigé vers l'afficheur et vers l'enregistreur.

Le stimulateur peut délivrer une impulsion unique, une série rapide d'impulsions ou un nombre prédéterminé d'impulsions à intervalles de temps réguliers mais n'est pas capable de fournir d'autres formes d'ondes que des rectangles (par exemple de 200 µs de durée).

Ce système comporte un module destiné à gérer le retard entre le stimulateur et la chaîne d'acquisition afin de contrôler le début de l'intégration. Il n'y a pas de déclenchement par niveau.

La demande de brevet GB-A-2 113 846 décrit un système de contrôle de l'état d'un patient lors d'une anesthésie réalisant une mesure d'EEG *(électroencéphalogramme)* couplée à des mesures d'EMG. Un système de stimulation-réponse permet de mesurer le taux de relâchement musculaire en stimulant un nerf moteur et en mesurant la réponse du muscle correspondant. Les courbes sont enregistrées et affichées en temps réel.

Il n'y a pas de description du système de stimulation-réponse, ni de la méthode de mesure pour évaluer le taux de décontraction musculaire. On utilise un "train de quatre" *(Train of Four,* TOF), c'est-à-dire quatre impulsions électriques séparées d'une demi-seconde. Pour chaque impulsion électrique, le système affiche une barre d'histogramme représentant la surface (intégrale) de l'EMG redressé correspondant. Le système comprend un convertisseur analogique/numérique (CAN) et une mémoire pour stocker les informations affichées

Le brevet américain US-A-4 595 018 décrit une méthode logicielle pour mesurer la transmission neuromusculaire (NMT). Les mesures sont entachées d'un artefact de stimulation. Pendant la phase de calibrage, l'appareil effectue une première mesure et mémorise la valeur de l'artefact de stimulation. En numérisant les informations collectées, l'erreur sur la mesure est éliminée mathématiquement. Cette méthode présente le désavantage que l'artefact perturbe malgré tout la chaîne de mesure.

Le document KR-A-9 004 899 décrit un appareil comprenant un capteur EMG pour la détection d'un signal électrique le long d'un tissu musculaire, un amplificateur EMG pour le filtrage et l'amplification du signal détecté, un afficheur, un contrôleur du signal pour l'analyse en temps réel du signal EMG et pour la réalisation de calculs à grande vitesse sur le signal, ainsi qu'un stimulateur pour la transmission d'un signal stimulus à la surface du tissu musculaire.

Le brevet US-A-5 300 096 décrit un stimulateur musculaire électrique permettant d'obtenir des signaux électromyographiques (EMG) numériques pour l'analyse et l'affichage par un programme d'ordinateur. Le système travaille par stimulation-réponse, c'est-à-dire excitation électrique d'un nerf moteur suivie par l'acquisition du signal EMG associé.

Le stimulateur délivre un train d'impulsions thérapeutique pré-sélectionné. Un circuit d'interconnexion permet de relier le système d'acquisition déclenchable par niveau, le stimulateur et l'ordinateur. Le software permet de gérer la période, l'intensité, etc., des impulsions électriques, le seuil de déclenchement du système d'acquisition et l'affichage en temps réel.

Le principe du système est similaire à celui décrit dans le brevet US-A-4 291 705 mais sous forme numérique, ce qui nécessite un PC.

Le brevet US-A-6 224 549 décrit un système de monitoring médical pour signaux électrophysiologiques de différents types (EEG, EMG, EP ou *Evoked Potential)* comprenant :

- un ordinateur pour l'affichage et la mémorisation de la courbe ;
- une interface utilisateur implantée dans le PC.

Ce système stocke la réponse sur le disque dur et l'affiche. L'interface utilisateur comporte un jeu de panneaux qui permettent d'afficher un ou plusieurs signaux. On dispose d'un certain nombre d'options d'affichage (trigger, calcul de la moyenne, analyse spectrale). Le signal affiché peut être généré à partir d'un stimulateur auditif, visuel ou électrique. Le système adapte la bande passante en fonction du type de signal mesuré (EMG, EEG, ...) .

Ce document ne donne pas de description de la chaîne d'acquisition et du stimulateur. Le brevet porte essentiellement sur le software (interface utilisateur). Un désavantage est la nécessité de disposer d'un PC, d'où perte de portabilité, sécurité, etc. (voir section 1.7).

L'utilisateur peut choisir le type de stimulateur (électrique, auditif ou visuel).

La demande internationale WO-A-02 053012 décrit un appareil digital d'électrodiagnostic clinique pour la mesure d'électromyographie (EMG) et/ou de conduction nerveuse. Le système comporte une paire d'électrodes de mesure, un amplificateur, un filtre et un stimulateur. Le stimulateur et la chaîne d'acquisition sont connectés à l'entrée stéréo d'une carte multimédia intégrée à un PC. Il existe une isolation galvanique entre le PC et la chaîne d'acquisition. Le système est déclenchable par niveau. Un désavantage est également la nécessité de disposer d'un PC, d'où perte de portabilité, sécurité, etc. (voir section 1.7).

La demande internationale WO-A-99 41682 décrit un programme s'exécutant sur un PDA et permettant d'entrer des données relatives à un patient. Le PDA est également capable de se connecter à un réseau d'ordinateurs pour échanger des informations avec une base de données. Le système comporte une interface qui permet de collecter des signaux sur le patient.

Dans *"MIThril 2003 : Applications and Architecture", Rich De Vaul, Michael Sung, Jonathan Gips, Alex "Sandy" Pentland, Media Laboratory, Massachusetts Institute of Technology,* on décrit l'utilisation d'un PDA pour mesurer des signaux de type "biopotentiels" et communiquer avec un ordinateur central via une liaison sans fil. Cet article traite surtout de l'aspect informatique et réseau du système qui permet de faire des mesures telles que : EMG, ECG *(électrocardiogramme),* EEG, température, accélération, etc.

Dans *"Biosignals Offer Potential for Direct Interfaces and Health Monitoring", Vince Stanford, Pervasive Computing, IEEE, January-March 2004, pp. 99-103,* on traite également de l'acquisition de signaux électrophysiologiques à l'aide d'un PDA sur n'importe quelle partie du corps. Le PDA peut établir une communication sans fil avec un ordinateur central et envoyer les résultats de la mesure à distance.

Dans *"Mobile biosignal acquisition and processing on the Pocket PC", g.MOBIlab (www.gtec.at/products/ g.MOBIlab/gMOBIlab.htm),* on décrit un système permettant l'acquisition d'EEG, EMG, ECG, de pulsations cardiaques, etc., à l'aide d'un Pocket PC. ce système permet :

- la visualisation et l'enregistrement de jusqu'à 8 canaux simultanément ;
- le transfert des données (bio-signaux) à l'hôpital via GSM ou WAN ;
- la détermination de la position du sujet grâce à un module GPS.

Ce système est notamment utilisé pour mesurer l'effet des hautes altitudes sur les paramètres des EEG et des ECG.

Dans "*A Wireless Multi-Channel Data Acquisition System for Physiological Signals*", *J. Stefan Karlsson, Tomas Bäcklund, Urban Edström, Department of Biomédical Engineering and Informatics, University Hospital, Umea, Sweden and Centre for Biomedical Engineering and Physics, Umea University, Sweden,* un module spécifique conditionne le signal d'entrée, effectue une CAN du signal et le transmet au PC via une connexion sans fil (Bluetooth). Le software à bord du PC permet de collecter les données en temps réel, de visualiser les réponses et de stocker les échantillons. Le système, basé sur un système de transmission sans fil, est suffisamment performant pour permettre l'acquisition de tous les signaux électrophysiologiques.

*Appareils commerciaux*

On trouve dans le commerce des instruments permettant de réaliser des mesures EMG.

DATAQ Instruments Inc. (Akron, Ohio) propose une chaîne d'acquisition modulaire (hardware et software) comprenant un préamplificateur faible bruit permettant le conditionnement de signaux électrophysiologiques, en particulier EMG.

Cet appareil présente le désavantage d'un réglage manuel du gain d'amplificateur et de ne pas comporter de stimulateur.

MEGA Electronics Inc. (New Brunswick, New Jersey) propose un appareil pratique et portable que le travailleur peut emmener sur lui pour mesurer des informations telles que :

- l'importance de l'effort physique nécessaire au maintien d'une position particulière ;
- les phases de la tâche professionnelle qui impliquent une charge statique ;
- le niveau des pics de charge ;
- le développement éventuel d'une fatigue musculaire lors de l'accomplissement de l'activité ;
- une comparaison entre les tâches ;
- une comparaison entre les individus.

Les signaux peuvent être envoyés immédiatement par une connexion filaire vers un ordinateur pour une analyse plus approfondie, ou bien être enregistrés afin d'éviter l'emploi d'un câble de connexion entre l'opérateur et l'ordinateur.

Cet appareil présente la caractéristique unique que la synchronisation avec des images vidéo digitales est possible. De cette façon, on peut examiner précisément quelle position implique une charge musculaire statique.

Les désavantages de cet appareil sont l'absence de stimulateur, de liaison sans fil et d'affichage sur l'appareil, ainsi que le manque d'autonomie.

En anesthésie, on utilise spécifiquement des appareils basés à la fois sur :

- l'observation visuelle de la contraction (par ex. TOF-Watch®, Organon, Akzo Nobel). Un premier inconvénient de cet appareil est que la mesure est qualitative ;
- la mécanomyographie : utilisation d'une jauge de contrainte et mesure de force. L'inconvénient réside ici dans la difficulté de calibration. D'où, comme F = m.a, on recourt à une mesure d'accélération (indirecte) ;
- l'accélérométrie : mesure indirecte de l'activité musculaire par des mesures d'accélération. Les inconvénients sont que la mesure ne peut être réalisée que sur la main et qu'elle est en outre subjective et peu reproductible.

Ainsi, dans l'appareil TOF-Watch®, le stimulateur permet de fournir les trains d'ondes habituellement utilisés en anesthésie (par ex. ST, TOF, TETANOS, DBS), un accéléromètre permet de mesurer l'accélération du pouce (mesure indirecte de la force) et l'appareil affiche le rapport entre l'accélération de la quatrième et de la première flexion.

**Buts de l'invention**

La présente invention vise à proposer une solution qui permette de s'affranchir des inconvénients de l'état de la technique.

En particulier, l'invention vise à fournir un appareil de mesure de signaux électrophysiologiques de type EMG consécutifs à une électro-stimulation, qui soit portable, autonome, très peu encombrant, fiable, flexible, simple d'utilisation, conforme aux normes de sécurité électriques (limitation du courant de défaut) et de fabrication peu coûteuse.

Un but complémentaire de l'invention est de fournir un appareil qui puisse effectuer des mesures fiables malgré la diminution d'amplitude des signaux EMG au cours de la curarisation lors d'une anesthésie.

Un autre but de l'invention est de permettre un contrôle aisé et automatique de la pose correcte des électrodes de mesure.

Un autre but de l'invention est de permettre un calibrage rapide de l'appareil, eu égard à un éventuel artefact de stimulation, ainsi qu'une détermination précise de l'amplitude de l'excitation supra-maximale.

Un but encore complémentaire de l'invention est de fournir un appareil capable d'être relié ou piloté par un (réseau d') ordinateur(s) distant(s), éventuellement grâce à une connexion sans fil.

## Principaux éléments caractéristiques de l'invention

Un premier objet de la présente invention se rapporte à un appareil intégré, portable et autonome pour la mesure directe, l'affichage, le traitement et la transmission à distance de signaux électromyographiques (EMG) décrit selon les termes de la revendication 1. L'innovation réside dans l'adaptation automatique du gain d'amplification du signal EMG mesuré en combinaison avec le traitement de l'artefact inévitable de stimulation, de manière à optimiser l'usage de la résolution du convertisseur analogique/numérique du système. En d'autres termes, l'invention fournit un contrôle automatique de gain avec une précision maximale (c'est-à-dire une erreur relative de quantification minimale).
Une utilisation de prédilection de cet appareil est l'évaluation du taux de relâchement musculaire au cours de la curarisation pratiquée lors d'une anesthésie. La résolution est conservée, même lorsque l'amplitude du signal EMG diminue au cours du temps, comme c'est le cas dans cette application. De plus, l'invention contribue à résoudre le problème de la diminution du rapport signal-sur-bruit liée à la diminution de l'amplitude du signal EMG lors de la curarisation. De manière plus générale, l'invention permet une mesure efficace dans un environnement bruité (pollution électromagnétique).
Des formes d'exécutions préférées de l'invention sont détaillées dans les revendications subsidiaires 2 à 21.
Un second objet de la présente invention est décrit dans les revendications 22 et 23 qui concernent un procédé pour ajuster automatiquement le gain appliqué au signal d'entrée et conserver la résolution maximale du convertisseur analogique/numérique dans l'appareil de mesure susmentionné, tout en supprimant ou en atténuant l'artefact de stimulation, selon que cet appareil est utilisé respectivement en mode de déclenchement synchronisé sur la stimulation ou en mode de déclenchement par niveau.

## Brève description des figures

La figure 1 représente le schéma bloc du système de mesure selon l'invention.
La figure 2A représente schématiquement une forme de signal triangulaire.
La figure 2B représente schématiquement le paramétrage d'une séquence de stimulation.
La figure 3 représente le schéma de principe de la chaîne d'acquisition selon l'invention.
La figure 4 représente schématiquement le procédé d'ajustement automatique du gain.
La figure 5 représente schématiquement un signal EMG avec artefact de stimulation tel que $V_{EMG} > V_{ARTEFACT}$.
La figure 6 représente schématiquement un signal EMG avec artefact de stimulation tel que $V_{EMG} < V_{ARTEFACT}$.
La figure 7 montre la saturation de l'amplificateur suite à une amplification trop importante de l'artefact de stimulation.
La figure 8 montre l'ajustement du gain pour un EMG avec artefact de stimulation tel que $V_{ARTEFACT} > V_{EMG}$.
La figure 9 montre le schéma bloc détaillé de la chaîne d'acquisition.
La figure 10A représente schématiquement la mise en court-circuit des électrodes de mesure.
La figure 10B représente schématiquement le signal EMG avec artefact de stimulation et mise en court-circuit des électrodes de mesure (acquisition synchronisée sur la fin de la stimulation).
La figure 10C correspond au signal de la figure 10B selon que le système fonctionne en mode synchronisé ou en mode de déclenchement par niveau.
Les figures 10D et 10E montrent schématiquement le déclenchement de l'acquisition lors du dépassement d'un seuil de tension programmable indépendant du stimulateur.
La figure 10F montre schématiquement le déclenchement de l'acquisition à la demande de l'utilisateur (acquisition continue).
La figure 11 représente schématiquement la mise en réseau pour une mesure polytopique.
La figure 12 représente schématiquement une acquisition en boucle ouverte.
La figure 13 représente schématiquement une acquisition en boucle fermée, en salle d'opération.
La figure 14 représente graphiquement une recherche d'intensité menant à une excitation "supra-maximale".
La figure 15 représente l'amplitude du signal EMG en fonction de l'intensité des impulsions électriques.

## Description d'une forme d'exécution préférée de l'invention

### 1. Présentation de l'appareil

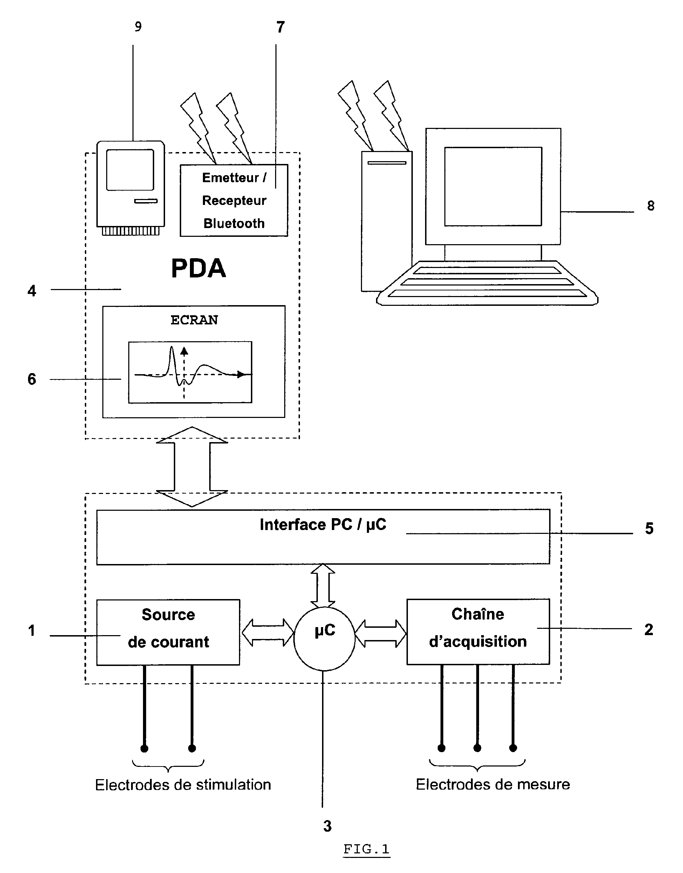

Le système selon l'invention illustré schématiquement sur la figure 1, comporte une source de courant 1 permettant l'excitation d'un nerf moteur périphérique et une chaîne d'acquisition 2 spécialement adaptée à la mesure des potentiels électromyographiques évoqués.

Au coeur du système on trouve un microcontrôleur 3 chargé d'assurer le pilotage et la synchronisation des différents modules du système en temps réel ainsi que la communication avec l'ordinateur 4 via une interface standardisée 5 (RS-232 , USB, RS-485, etc.).

Le signal EMG et/ou ses paramètres peuvent être visualisés sur l'écran d'affichage 6.

Bien que le système puisse être piloté à partir de n'importe quelle station de travail comportant un port de communication standard (RS-232, USB, RS-485, etc.), il convient d'utiliser de préférence un PDA *(Personal Digital Assistant)* 4 pour réaliser l'interface utilisateur afin d'assurer la portabilité, l'autonomie et la sécurité du système (voir ci-après, section 1.7).

Afin de faciliter son intégration dans un système de monitoring médical, le système est capable d'établir une communication sans fil avec un ordinateur central 8 via un émetteur/récepteur "Bluetooth" 7 intégré dans le PDA.

Les réponse EMG peuvent être enregistrées dans la mémoire non-volatile 9 disponible à bord du PDA (SD, CompactFlash, etc.).

L'invention comprend également un programme 6 servant d'interface à l'utilisateur et permettant d'exploiter les données provenant du système embarqué.

 L'utilisateur peut choisir :

- le mode d'acquisition du système :

  - mode synchronisé ;
  - mode déclenché par niveau ;
  - mode continu.

Chacun de ceux-ci définit une configuration différente du système et une gestion différente de la synchronisation entre les modules ;

- le mode d'affichage de la courbe réponse ;
- le type de stimulation à délivrer ; etc.

La suite de la description est consacrée à chacun des modules cités ci-dessus et met en évidence les avantages qui résultent du regroupement de ces modules dans un même appareil.

### 1.1 Le stimulateur

1.1.1 Signaux délivrés

Le système comporte un stimulateur pouvant travailler dans deux modes différents. Dans le premier mode, le stimulateur délivre des séquences de stimulation programmables par l'utilisateur. Dans le second, il délivre les trains d'impulsions habituellement utilisés en anesthésie.

Mode programmé

L'électro-stimulateur contient une série de séquences d'impulsions rectangulaires préenregistrées dans sa mémoire, tels que le ST (Single Twich), le TOF (Train Of Four), le TS (Tetanic Stimulation) ou le DBS (Double Burst Stimulation).

L'intensité, la largeur des impulsions et la période entre deux séquences successives ont une valeur par défaut mais sont reparamétrables par l'utilisateur dans la gamme :

- amplitude des impulsions : 0 - 150 mA dans 5 k$\Omega$ ;
- largeur des impulsions : 0 - 1000 $\mu$s ;
- période séparant deux séquences successives : 10 - 60 s.

Mode programmable

L'utilisateur peut choisir à la fois la forme du signal (sinusoïdale, triangulaire, rectangulaire, de forme arbitraire), sa fréquence et son amplitude.

Rectangulaire :

- amplitude : 0 - 100 mA dans 5 kΩ ;
- largeur des impulsions : 0 - 1000 $\mu$s.

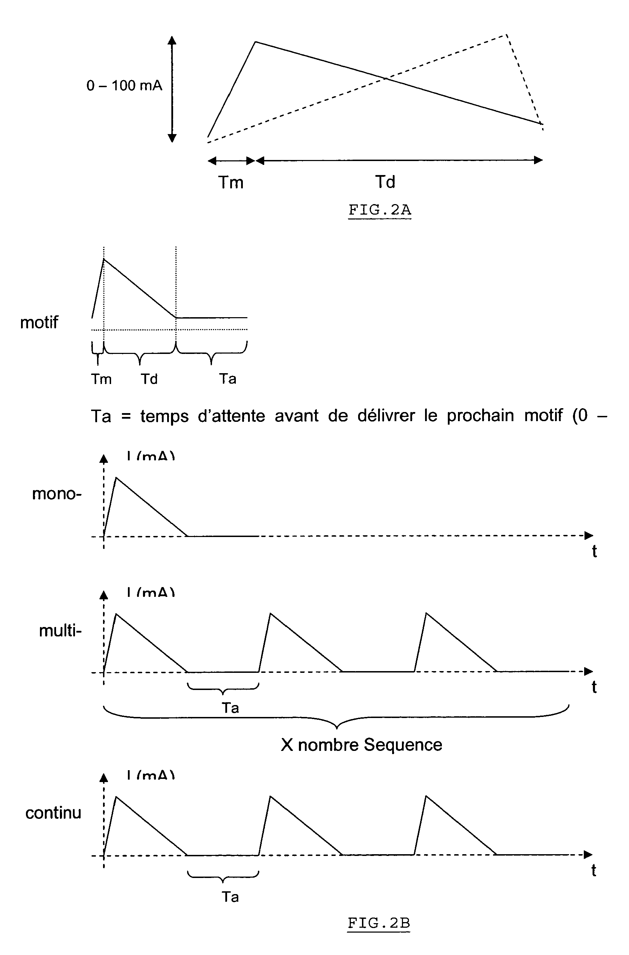

Triangulaire (voir figure 2A) :

- amplitude : 0 - 100 mA dans 5 kΩ;
- temps de montée (Tm) : 15 $\mu$s - 5 ms ;
- temps de descente (Td) : 15 $\mu$s - 5 ms.

Sinusoïdale :

- amplitude : 0 - 100 mA ;
- fréquence : 0 - 200 Hz.

Une fois le motif établi, l'utilisateur peut choisir de travailler en mode :

- *mono-impulsion :* pour délivrer un motif unique ;
- *multi-impulsion* : pour délivrer un nombre déterminé de motifs en fixant le temps "$T_a$" entre deux motifs consécutifs ;
- *continu* : pour électro-stimuler en continu en fixant le temps "$T_a$" qui sépare deux motifs consécutifs.

La figure 2B illustre le paramétrage d'une séquence de stimulation.

1.1.2 Aspects sécurité

Le stimulateur effectue régulièrement ou à la demande la mesure d'impédance entre les électrodes de stimulation selon les normes médicales pour le courant de défaut.

**1.2 La chaîne d'acquisition**

1.2.1 Schéma de principe

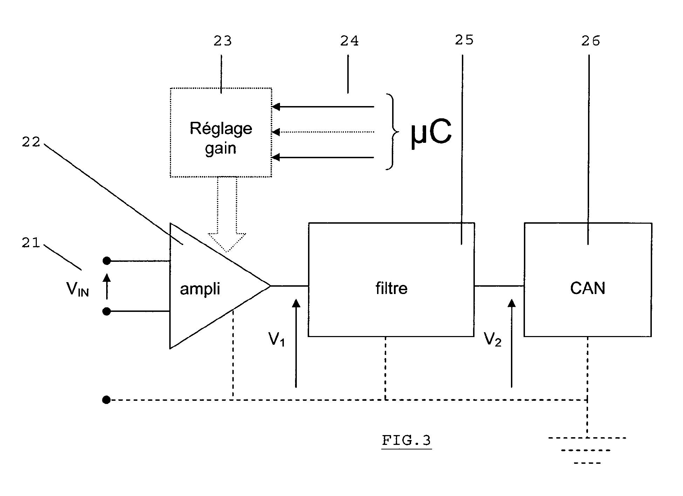

La chaîne d'acquisition 2, représentée schématiquement sur la figure 3, a pour rôle d'amplifier le signal provenant des électrodes de mesure 21 en "$V_1$" à l'aide d'un amplificateur différentiel 22, de le filtrer à travers un filtre 25 pour en extraire les fréquences indésirables et d'effectuer la conversion analogique/numérique 26 du signal "$V_2$" ainsi conditionné.

Pour assurer une résolution maximale dans la conversion analogique numérique 26, le système de réglage automatique du gain 23 commandé 24 à partir du microcontrôleur permet d'amplifier le signal d'entrée 21 afin d'exploiter au mieux la plage de tension du convertisseur.

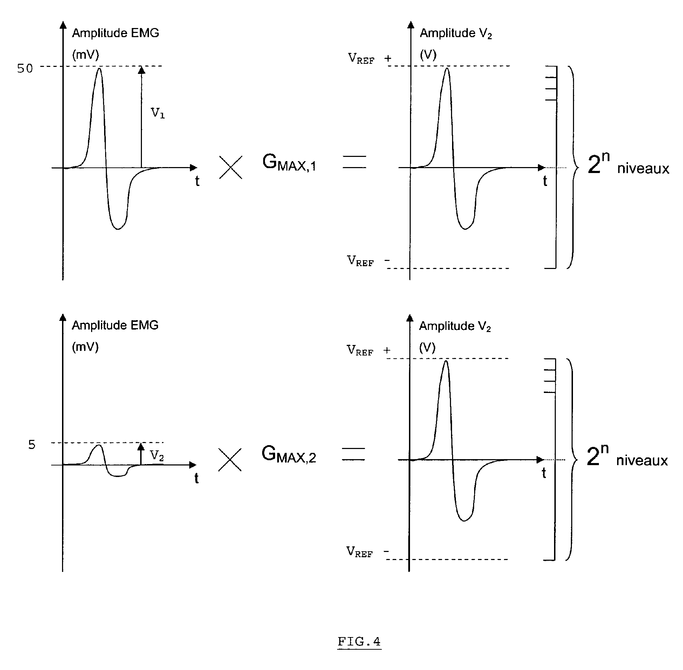

En choisissant comme gain (voir figure 4):

$$G_{MAX} = \frac{V_{REF}}{V_{MAX}} \quad (1),$$

où :

- $V_{REF}$ est la demi plage d'entrée du convertisseur analogique numérique et
- $V_{MAX}$ est la valeur de crête du signal à mesurer, l'amplitude du signal "$V_2$" à l'entrée du convertisseur exploitera la "totalité" de sa plage de tension. Toujours selon la figure 4, on a :

$$G_{MAX,1} = \frac{V_{REF}}{V_1} \,,$$

$$G_{MAX,2} = \frac{V_{REF}}{V_2} \, ,$$

avec

$$V_{REF} = \max( \left| V_{REF\,+} \right| \, , \, \left| V_{REF\,-} \right| ) \, .$$

1.2.2 <u>Problème lié à l'artefact de stimulation</u>

La stimulation provoque un artefact gênant pour la mesure d'EMG de faible amplitude.

Les signaux EMG étant d'amplitude relativement faible et collectés dans un environnement assez bruité, il convient de les amplifier au maximum et le plus près possible du site de mesure.

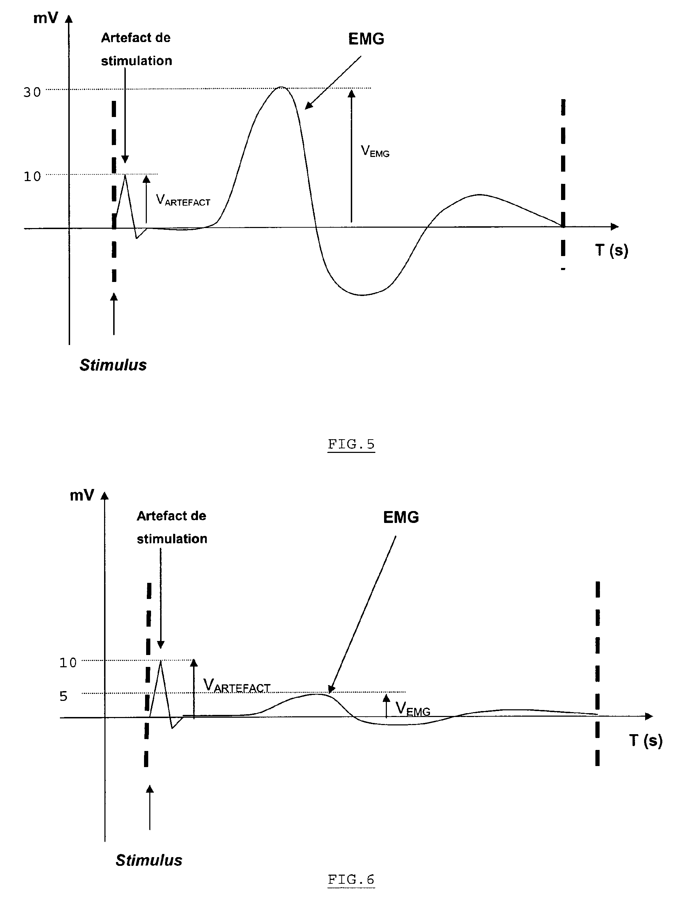

La figure 5 montre que, pour des EMG d'amplitude convenable (lorsque le patient n'est pas curarisé), l'amplitude de la réponse musculaire est généralement plus élevée que l'amplitude de l'artefact de stimulation.

La condition (1) et $V_{MAX} = V_{EMG}$ impliquent que

$$G_{MAX} = \frac{V_{REF}}{V_{EMG}} \quad (2) \, .$$

Le gain maximum de l'amplificateur est donc inversement proportionnel à l'amplitude du signal EMG.

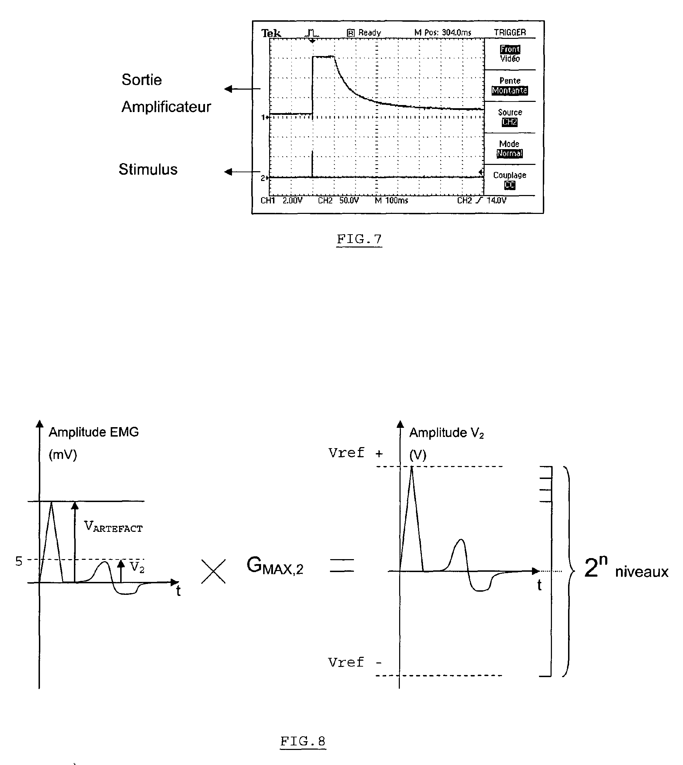

Comme l'augmentation de la concentration de curare provoque une diminution progressive et considérable de l'amplitude des EMG, il arrive un moment où l'amplitude de l'EMG devient inférieure à celle de l'artefact de stimulation (voir figure 6).

Se pose alors le choix du gain. Un dimensionnement similaire à celui de l'expression (2) basée sur l'amplitude de l'EMG pourrait provoquer une saturation de l'amplificateur suite à une amplification excessive de l'artefact de stimulation, comme cela est montré sur la figure 7.

Le gain maximum de l'amplificateur ne dépend donc plus de l'amplitude du signal EMG mais bien de l'amplitude de l'artefact de stimulation, ce qui entraîne un mauvais rapport signal-sur-bruit (SNR ou *Signal-to-Noise* Ratio) pour les petits EMG.

La figure 8 montre l'ajustement du gain pour un EMG avec artefact de stimulation tel que $V_{ARTEFACT} > V_{EMG}$.

On utilise deux méthodes, selon le mode d'utilisation, pour minimiser l'influence de l'artefact sur le signal mesuré et qui permettent d'exploiter de manière utile toute la plage d'entrée du CAN.

Si $V_{ARTEFACT} > V_{EMG}$, alors

$$G_{MAX} = \frac{V_{REF}}{V_{ARTEFACT}} \quad (3) \, ,$$

et si $V_{EMG} > V_{ARTEFACT}$, alors

$$G_{MAX} = \frac{V_{REF}}{V_{EMG}} \quad (4) \, ,$$

où

- $V_{EMG}$ est l'amplitude crête de l'EMG,
- $V_{ARTEFACT}$ est l'amplitude crête de l'artefact,
- $V_{REF}$ est la demi-plage d'entrée du CAN.

1.2.3 <u>Solutions mises en oeuvre</u>

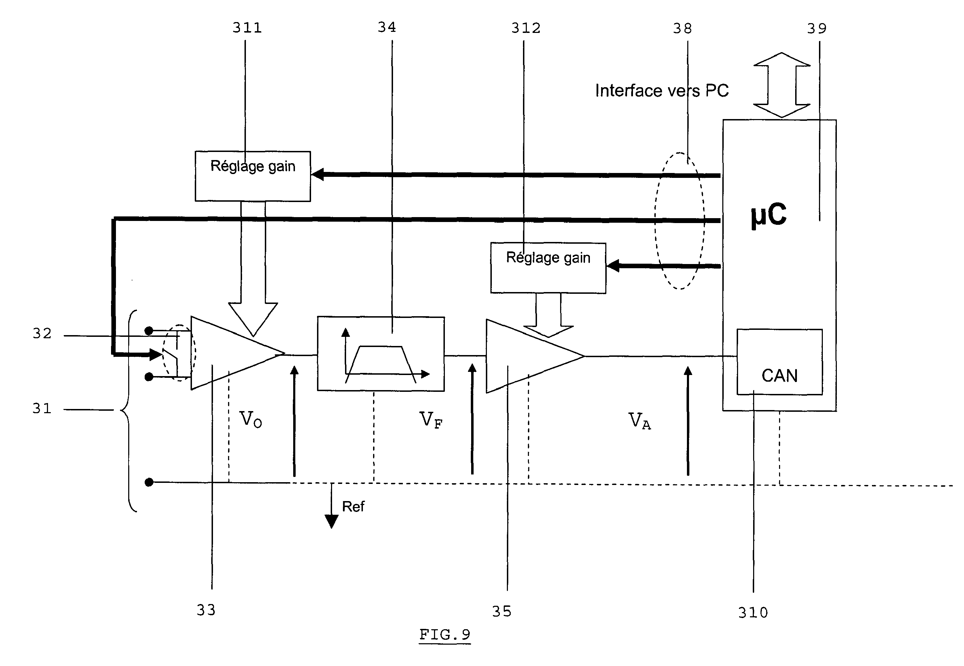

Sur la gauche du schéma-bloc représenté sur la figure 9, on peut distinguer le connecteur 31 vers les électrodes de mesure et l'électrode de référence. Le système effectue une préamplification différentielle 33 afin de minimiser les bruits de mode commun captés par le corps humain.

Entre le préampli 33 et le connecteur pour les électrodes 31, on peut distinguer la présence d'un relais 32, piloté par une sortie 38 du microcontrôleur 39, permettant la mise en court-circuit des électrodes de mesure.

Le signal préamplifié "$V_0$" passe à travers un filtre passe-bande 34 pour ne conserver que les fréquences utiles de celui-ci (10 - 1000 Hz).

Le signal filtré "$V_F$" peut éventuellement être réamplifié 35 afin de s'inscrire au mieux dans la plage de tension d'entrée du convertisseur analogique numérique 310 (voir "mode 2" ci-après).

Deux modules distincts 311 et 312 permettent d'ajuster indépendamment le gain du préamplificateur 33 et celui de l'amplificateur 35 via certaines pattes de sortie 38 du microcontrôleur 39.

**a) mode de déclenchement synchronisé sur la stimulation (mode 1)**

Dans ce cas, on procède à un masquage du signal. Cette méthode consiste à court-circuiter les électrodes d'acquisition pendant la durée de l'artefact de stimulation. Elle nécessite que le stimulateur soit couplé à la chaîne d'acquisition EMG et qu'il fournisse un signal de synchronisation.

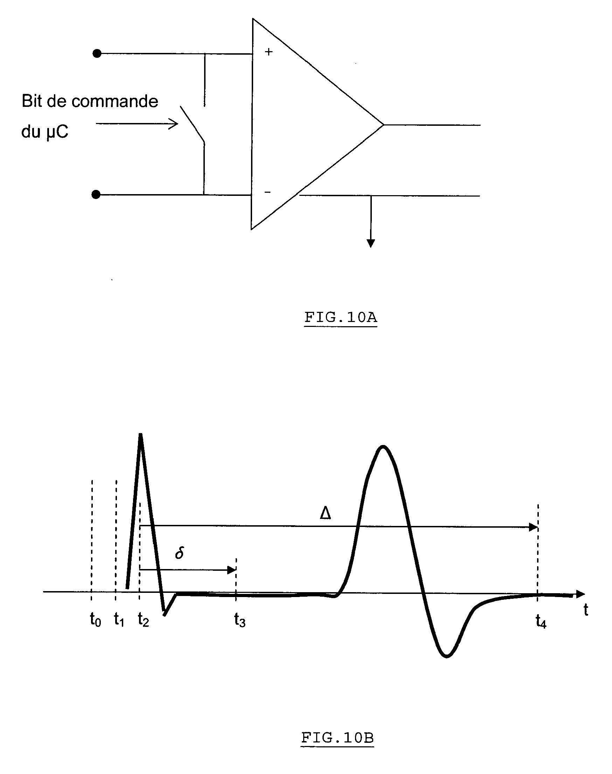

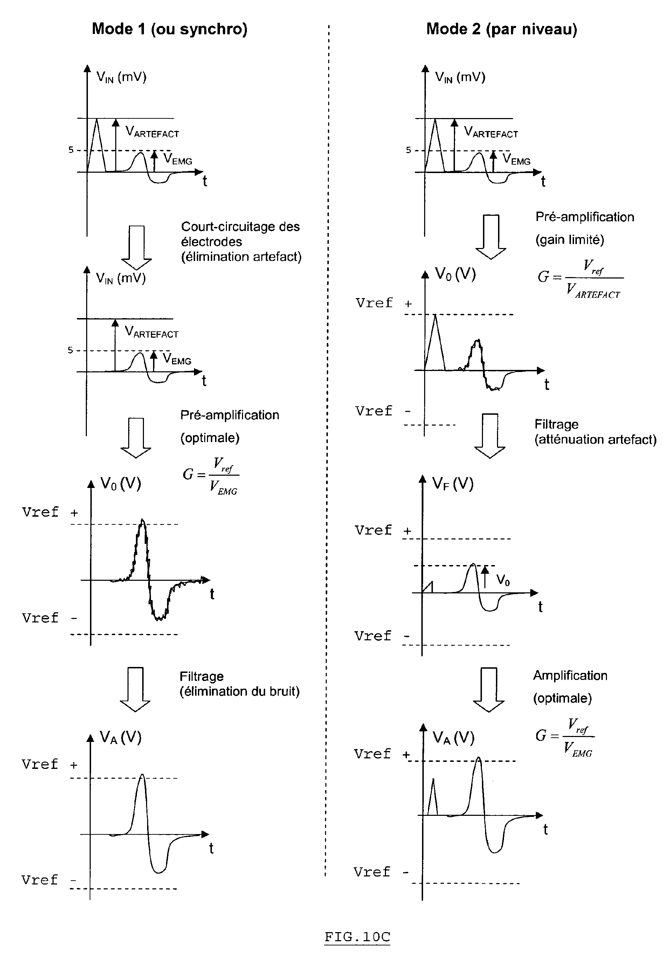

La figure 10A montre le principe du court-circuitage des électrodes de mesure par le microcontrôleur. La figure 10B montre le signal obtenu, où :

- $t_0$ est l'instant de court-circuitage des électrodes de mesure ;
- $t_1$ est l'instant de début de la stimulation ;
- $t_2$ est l'instant de fin de la stimulation et du début de l'acquisition ;
- $t_3$ est l'instant de décourt-circuitage des électrodes de mesure ;
- $t_4$ est l'instant de fin d'acquisition ;
- $\delta$ est la durée de court-circuitage des électrodes après la stimulation et
- $\Delta$ est la période d'enregistrement.

De cette manière, l'artefact de stimulation a totalement disparu du signal mesuré et la condition $V_{EMG} > V_{ARTEFACT}$ est constamment vérifiée.

Le gain du préamplificateur peut être dimensionné immédiatement de manière optimale. Le délai entre la fin de la stimulation et l'ouverture du relais court-circuitant les électrodes est rendu programmable par l'utilisateur.

**b) <u>mode de déclenchement par niveau ou continu (mode 2)</u>**

Dans ce cas, l'amplification est bi-étagée avec filtrage intermédiaire de l'artefact de stimulation.

Lorsque la chaîne d'acquisition ne peut pas recevoir de signal de synchronisation, elle doit fonctionner en mode de déclenchement par niveau (voir 1.3.2). Il est impossible de court-circuiter les électrodes de mesure pendant la stimulation. Le réglage du gain de l'amplificateur dépend des conditions (3) et (4) établies précédemment, soit :

- Si $V_{EMG} > V_{ARTEFACT}$, alors

$$G_{MAX} = \frac{V_{REF}}{V_{EMG}} \, ,$$

ce qui est la condition optimale ;
- si $V_{ARTEFACT} > V_{EMG}$, alors

$$G_{MAX} = \frac{V_{REF}}{V_{ARTEFACT}} \; ,$$

avec augmentation de l'erreur de quantification.

La solution mise en oeuvre consiste alors à :

- effectuer une préamplification du signal à mesurer en respectant la condition (3), soit

$$G_{MAX,1} = \frac{V_{REF}}{V_{ARTEFACT}} \; ,$$

avec

$$V_0 = G_{MAX,1} \cdot V_{EMG} \quad ;$$

- filtrer ce signal afin de ne conserver que la bande d'énergie utile du signal. L'artefact de stimulation, se trouvant en dehors de cette bande d'énergie, aura été suffisamment atténué pour que son amplitude redevienne inférieure à celle de l'EMG. De cette manière, la condition (4) est de nouveau vérifiée et le signal pourra être réamplifié de manière optimale ;
- amplifier le signal filtré en respectant la condition (4), soit

$$G_{MAX,2} = \frac{V_{REF}}{V_0} \; .$$

La comparaison des différentes étapes de traitement dans les modes 1 et 2 est illustrée sur la figure 10C.
De cette manière, que le système fonctionne en mode synchronisé (avec court-circuitage des électrodes de mesure) ou en mode de déclenchement par niveau (sans court-circuitage des électrodes de mesure) et quelle que soit l'amplitude relative de l'EMG par rapport à l'artefact de stimulation, nous pouvons toujours amplifier le signal de manière optimale, c'est-à-dire en maintenant constante l'erreur relative de quantification.

### 1.3 Modes de fonctionnement de l'appareil

Le système est conçu pour fonctionner dans trois modes différents et son architecture est adaptée pour maintenir constante l'erreur de quantification dans chacun des modes.

#### 1.3.1 Mode 1 ou mode de déclenchement synchronisé

L'acquisition est synchronisée sur la fin de la stimulation. Les électrodes sont court-circuitées pendant un temps $\delta$ programmable (1 - 1000 $\mu$s) par rapport à la fin de la stimulation, comme représenté à la figure 10B, avec :

- $t_0$ est l'instant de court-circuitage des électrodes de mesure ;
- $t_1$ est l'instant de début de la stimulation ;
- $t_2$ est l'instant de fin de la stimulation et du début de l'acquisition ;
- $t_3$ est l'instant de décourt-circuitage des électrodes de mesure ;
- $t_4$ est l'instant de fin d'acquisition ;
- $\delta$ est la durée de court-circuitage des électrodes après la stimulation, programmable de 1 à 1000 $\mu$s et
- $\Delta$ est la période d'enregistrement, programmable de 1 à 10000 ms.

La source de courant et la chaîne d'acquisition étant pilotée par le même microcontrôleur, il est aisé de synchroniser le début de l'acquisition sur la fin de la stimulation.

Une sortie du microcontrôleur pilote un relais qui permet de court-circuiter les électrodes de mesure pendant les premières microsecondes qui suivent la stimulation, ce qui a pour effet de masquer l'artefact de stimulation et ce qui autorise l'augmentation du gain des amplificateurs sans risque de saturation.

Grâce à cette méthode, la mesure du potentiel électromyographique évoqué s'effectue toujours dans les conditions optimales définies précédemment [voir relation (2)].

Il est donc possible d'optimiser le gain du préamplificateur et d'augmenter ainsi le rapport signal sur bruit pour les EMG de faibles amplitudes.

1.3.2 Mode 2 ou mode de déclenchement par niveau

Le déclenchement de l'acquisition a lieu lors du dépassement d'un seuil de tension programmable (indépendamment du stimulateur).

De cette manière, il est possible de mesurer des potentiels électromyographiques évoqués à partir d'un stimulateur externe ou même de mesurer des EMG spontanés (mouvements volontaires sans stimulation).

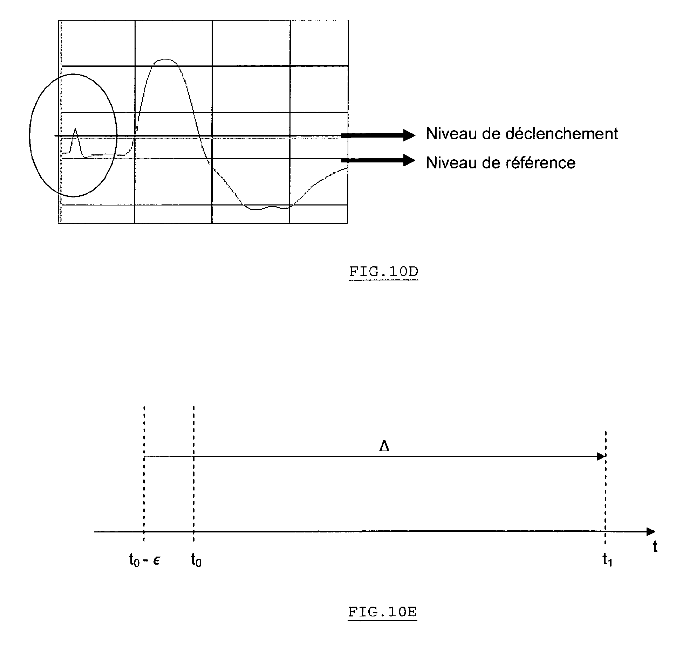

Le niveau de référence et le niveau de déclenchement sont indiqués schématiquement sur les figures 10D et 10E. Sur la figure 10E :

- $t_0$ est l'instant de dépassement du seuil de tension ;
- $t_0 - \varepsilon$ est l'instant du début de l'acquisition (buffer circulaire de taille programmable) ;
- $t_1$ est l'instant de fin d'acquisition et
- $\Delta$ est la période d'enregistrement, programmable de 1 à 10000 ms.

Un buffer (tampon) circulaire de taille programmable contient les échantillons précédant la condition de déclenchement.

Aucun court-circuitage des électrodes n'est possible, d'où la présence éventuelle de l'artefact de stimulation dans ce mode de fonctionnement.

Comme déjà mentionné (figure 10C), afin de conserver une résolution constante sur le CAN, le système effectue une amplification bi-étagée avec un filtrage intermédiaire de l'artefact de stimulation.

1.3.3 Mode 3 : déclenchement à la demande (acquisition continue)

Le déclenchement de l'acquisition se fait à la demande de l'utilisateur.

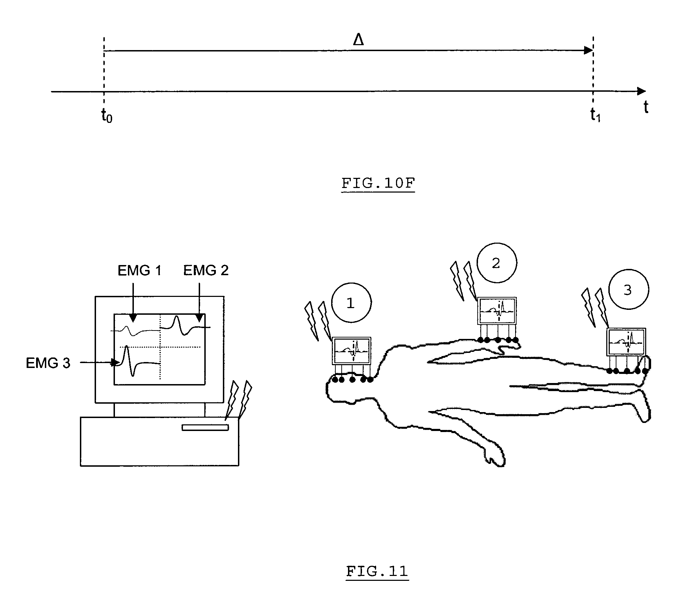

Le début de l'enregistrement correspond à la pression sur le bouton "REC" de l'interface utilisateur et la fin d'enregistrement correspond à la pression sur le bouton "STOP" de l'interface utilisateur. Le signal obtenu est représenté sur la figure 10F où :

- $t_0$ est l'instant de la pression sur le bouton "REC" ;
- $t_1$ est l'instant de la pression sur le bouton "STOP" et
- $\Delta$ est la période d'enregistrement.

## 1.4 Aspects sécurité

Le système selon l'invention est conçu pour réaliser régulièrement ou à la demande la mesure d'impédance aux niveaux des électrodes d'acquisition.

En effet, en anesthésie, il est impératif de distinguer entre la diminution de l'amplitude de l'EMG due aux effets du curare et celle due au décollement des électrodes de mesure.

Le système comporte des résistances de protection pour limiter lé courant de défaut dans le cas ou la tension d'alimentation serait appliquée accidentellement sur les électrodes de mesure.

## 1.5 L'interface utilisateur

1.5.1 Choix du mode de fonctionnement du programme

- Enregistrement :

Dans ce mode, le système effectue l'acquisition de l'EMG, sauvegarde la réponse sur la mémoire non-volatile, affiche la courbe en respectant le mode d'affichage sélectionné, effectue un traitement de la courbe et affiche les paramètres déterminants.

- Lecture :

Dans ce mode, l'utilisateur lit les EMG enregistrés. Le système effectue une lecture dans la mémoire de l'appareil, affiche le signal en respectant le mode d'affichage sélectionné, effectue un traitement de la courbe et affiche les paramètres déterminants.

1.5.2 Choix du mode de fonctionnement de l'appareil

L'interface évolue en fonction du mode sélectionné :

- en mode "Synchronisé", l'utilisateur règle le temps de court-circuitage des électrodes ;
- en mode "Déclenché par niveau", l'utilisateur règle le seuil de déclenchement (et éventuellement la pente) ;
- en mode "Continu", l'utilisateur règle le gain des amplificateurs et appuie respectivement sur les boutons "REC" et "STOP" pour démarrer et arrêter une acquisition.

1.5.3 Réglage des paramètres du stimulateur

Il s'agit du choix du mode : programmé ou programmable.
En mode programmé, l'utilisateur choisit le type de séquence à délivrer (ST, TOF, ...), l'intensité et la largeur des impulsions.
En mode programmable, l'utilisateur dessine ses propres formes d'onde à l'aide des outils graphiques appropriés mis à disposition.

1.5.4 Ajustement du gain

- Automatique :

Dans ce mode, l'ajustement du gain des amplificateurs est transparent à l'utilisateur. Le système adapte automatiquement le gain comme vu précédemment.

- Manuel :

L'utilisateur peut choisir à la fois le gain du pré-ampli et le gain de l'ampli de la chaîne d'acquisition.
1.5.5 Mode d'affichage
Les différents modes d'affichage habituels de l'état de la technique sont prévus.
1.5.6 Choix des paramètres critiques
L'évaluation du relâchement musculaire peut se faire notamment en mesurant le rapport de l'amplitude crête-à-crête de l'EMG curarisé sur l'amplitude crête-à-crête d'un EMG de référence (par exemple $T_1/T_0$, $T_4/T_1$, etc.) ou en mesurant le rapport des surfaces (par exemple $S_1/S_0$, $S_4/S_1$, etc.) des EMG redressés (c'est-à-dire les potentiels pris en valeur absolue). Toutefois beaucoup de paramètres peuvent être utilisés. Le logiciel permet de faire l'analyse complètement, aussi bien en temps réel qu'en post-traitement.

**1.6 Fonctionnalités complémentaires**

1.6.1 Emetteur/Récepteur "Wireless"

L'utilisation de la technologie "Wireless" entraîne :

- la possibilité de prise de mesure à distance (donc sans fil) et la gestion décentralisée de l'appareil ;
- la possibilité de mise en réseau pour mesure polytopique (voir figure 11).

L'appareil est conçu pour pouvoir travailler en "esclave" d'un ordinateur central via une connexion sans fil. L'ordinateur central est par ailleurs capable d'établir une communication avec plusieurs systèmes de stimulation-réponse EMG et de les interroger à tour de rôle (figure 11).

1.6.2 Sauvegarde des EMG

Les EMG sont enregistrés sur la carte mémoire du PDA.

**1.7 Avantages de l'invention**

En résumé, un certain nombre de caractéristiques avantageuses de l'appareil selon la présente invention permettent de distinguer cet appareil de l'état de la technique connu.
Masquage de l'artefact de stimulation

-  la chaîne d'acquisition n'est pas perturbée par l'artefact de stimulation ;
-  possibilité d'amplifier dans les conditions optimales quelle que soit l'amplitude de l'EMG ;
-  amélioration du rapport signal sur bruit.

Fonctionnement en maître ou en esclave - "Master/Slave"

-  le système peut travailler de manière complètement autonome (PDA maître) ;
-  le système peut travailler en esclave d'un ordinateur central pour effectuer des mesures à la demande (PDA esclave).

Liaison sans fil avec le PDA - "Wireless"
Le système utilise par exemple un émetteur/récepteur "Bluetooth" intégré dans le PDA pour communiquer avec un autre ordinateur.
Le programme s'exécutant sur le PDA est indépendant de l'OS

-  Operating System
-  programme écrit en Java ;
-  il suffit que le fabricant de PDA fournisse une JVM *(Java Virtual Machine)* adaptée au système d'exploitation ;
-  le programme peut s'exécuter sur la plupart des PDA ayant une puissance de calcul suffisante.

Portabilité
Elle est liée à la miniaturisation des composants :

-  possibilité d'effectuer des mesures sur n'importe quelle partie du corps, donc:

    -  compatible avec tous les muscles périphériques ;
    -  pas de limitation à la main ;

-  possibilité d'effectuer des mesures sur n'importe quel site géographique, donc utilisable en salle d'opération, au lit du patient, au cabinet du médecin, à domicile, etc.

Sécurité

-  le système de l'invention consomme un minimum ; entièrement sur batterie, il évite les problèmes liés à l'isolation galvanique ; ce système est particulièrement bien adapté à une mesure polytopique (pas de masse commune pour les différents capteurs) ;
-  le système comporte en outre des résistances de protection afin de répondre aux normes médicales qui imposent de limiter le courant de défaut à 50 $\mu$A.

Intérêt économique
Les composants de l'appareil sont peu coûteux.
Général
Puisqu'il s'agit d'une mesure électrique, l'appareil de l'invention convient chaque fois qu'on peut placer des aiguilles ou des électrodes. Il convient pour la main mais aussi pour n'importe quel autre site.
Fiabilité *(Reliability)*
Le système effectue un test de décollement des électrodes de stimulation et d'acquisition avant chaque campagne de mesure et avertit lorsque les électrodes de stimulation ou d'acquisition sont mal positionnées, par exemple lorsqu'une électrode est mal collée.
Electrodes

L'appareil convient aussi bien pour des électrodes de surface que pour des électrodes à aiguilles, des implants ou encore des électrodes actives, possédant leur propre préamplificateur incorporé.

Flexibilité (*Diversity*)

Programmabilité des stimuli élémentaires, de leur séquencement ou de leur répétition dans le temps :

- le stimulateur peut fournir des trains d'impulsions préenregistrées ;
- le stimulateur peut fournir des formes d'ondes dessinées par l'utilisateur.

Déclenchement *(Triggering)*

Le déclenchement de l'acquisition s'obtient dans trois modes différents :

- mode de déclenchement synchronisé : acquisition synchronisée sur la fin de la stimulation ; court-circuitage des électrodes de mesures pendant un temps δ programmable ;
- mode de déclenchement par niveau : dépassement d'un seuil de tension programmable ;
- mode de déclenchement à la demande : acquisition continue.

Contrôle automatique du gain

Le système ajuste automatiquement le gain des amplificateurs pour conserver la résolution lorsque l'amplitude des EMG diminue.

Résolution fixe (*Fixed Resolution*)

L'invention exploite astucieusement la grandeur du signal, en adaptant automatiquement l'amplification de la chaîne de mesure pour exploiter le maximum de la résolution du CAN du système.

Reproductibilité de la mesure

La reproductibilité est assurée dès qu'on a bien positionné les électrodes.

Autonomie

L'autonomie de l'appareil selon l'invention résulte de :

- la portabilité (volume) ;
- la sécurité (batterie) ;
- l'autonomie propre (maître).

Affichage - *Display*

Le système permet différents mode d'affichage.

Memoire

Le système assure l'enregistrement des réponses musculaires sur la carte mémoire.

Agenda

L'utilisateur peut programmer différents modes de stimulation et le moment auquel il veut les délivrer, ce qui est utile en salle d'opération par exemple (phase de curarisation, phase opératoire, phase de décurarisation).

Analyse complémentaire en post-traitement

L'utilisateur peut obtenir des informations complémentaires en post-traitement :

- amplitude crête-à-crête ;
- surface de l'EMG redressé ;
- analyse spectrale, etc. ;

ainsi que le rapport de certaines de ces mesures.

### 2. Domaines d'application

**2.1 Point de vue de celui qui administre les drogues (curare) et des neurophysiologistes**

L'appareil selon l'invention est tout d'abord utile pour apprécier l'effet de nouvelles molécules qui apparaissent sur le marché et dont il faut estimer les effets en différents sites, sur différents muscles.

En effet, d'une part, la constante de temps et l'inertie des effets des agents curarisants dépendent du type de drogue administrée au patient et, d'autre part, le taux de paralysie n'est pas uniforme dans toutes les parties du corps.

L'appareil décrit est également utile lors d'un examen neurophysiologique pour l'évaluation de la tonicité musculaire. On est souvent amené à apprécier le tonus musculaire d'un muscle donné ou la relation de paralysie ou de recouvrement entre deux muscles. En effet, lors d'une injection de curare, la paralysie du patient commence au niveau central et se termine par les muscles périphériques. De même, des muscles comme le diaphragme se paralysent avant les muscles situés aux extrémités, comme l'adducteur du pouce. Le processus de décurarisation s'effectue dans le même ordre.

Par exemple, si seul le pied est accessible, on voudrait pouvoir apprécier son tonus musculaire et par ailleurs connaître sa relation avec les tonus musculaires du larynx et du diaphragme pour savoir quand on peut intuber ou extuber un patient.

L'appareil de l'invention présente également la possibilité de mise en réseau pour une mesure polytopique (voir figure 11). L'appareil est conçu pour pouvoir travailler en esclave d'un ordinateur central via une connexion sans fil. L'ordinateur central est par ailleurs capable d'établir une communication avec plusieurs système de stimulation-réponse EMG et de les interroger à tour de rôle.

### 2.2 Point de vue des anesthésistes

Du point de vue des anesthésistes, l'appareil selon la présente invention est utile à deux titres :

- d'abord en salle d'opération pour apprécier le degré de bloc neuromusculaire lorsqu'on curarise un patient en boucle ouverte ou fermée (administration en bolus unique, répété, perfusion continue) ;
- ensuite pour évaluer la récupération de la fonction neuromusculaire d'un patient après une intervention chirurgicale, les groupes musculaires internes étant ceux qui sont impliqués dans la respiration et dans la protection des voies aériennes supérieures.

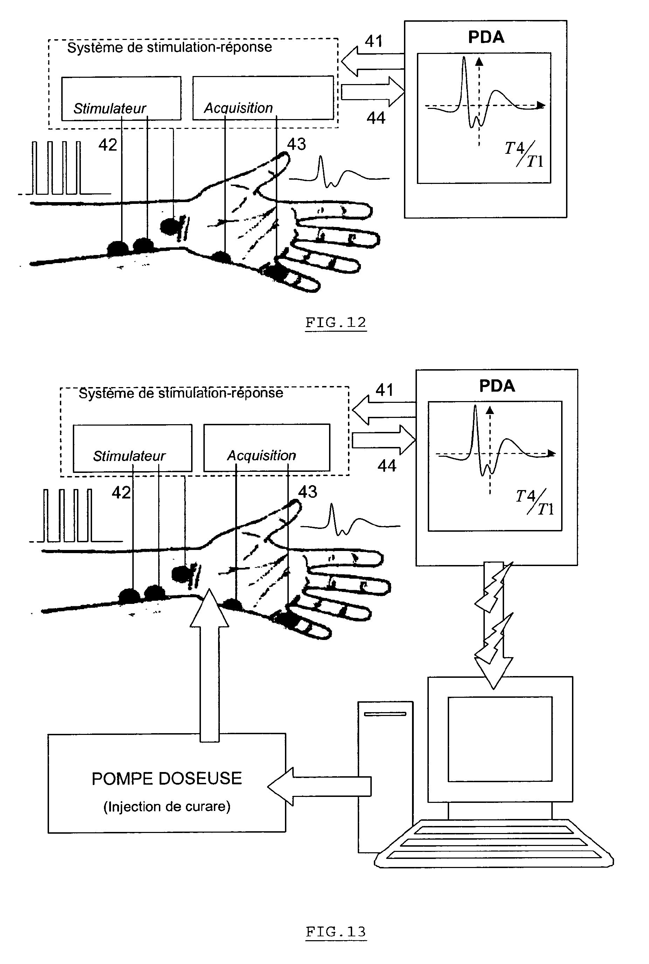

#### 2.2.1 Acquisition en boucle ouverte en salle d'opération (figure 12)

L'appareil fonctionne de manière complètement autonome et assure l'affichage de la courbe-réponse ainsi qu'une valeur chiffrée représentative de l'état de relâchement musculaire du patient ($T_4/T_1$ ou $T_1/T_0$, etc.).

L'utilisateur peut choisir de configurer le PDA pour établir un agenda des séquences de stimulation à délivrer durant l'intervention chirurgicale ou administrer des séquences de stimulation à la demande.

Lorsque l'on veut effectuer une mesure, une commande 41 est envoyée au microcontrôleur qui assure ensuite la gestion en temps réel.

Le stimulateur délivre le train d'impulsions sélectionné 42 sur un nerf moteur périphérique et le système d'acquisition mesure 43 la réponse du muscle correspondant. Le PDA reçoit ensuite la rafale de bytes 44 provenant du système et assure le traitement de la réponse (sauvegarde de la réponse, affichage de la courbe et évaluation des paramètres estimant le degré de bloc neuromusculaire).

Donc, le PDA fonctionne de manière complètement autonome et est le maître du système.

#### 2.2.2 Acquisition en boucle fermée en salle d'opération (figure 13)

Le PDA est capable de communiquer avec un ordinateur central via une connexion sans fil "Bluetooth" ou une connexion filaire avec isolation galvanique. L'appareil peut donc travailler comme esclave de l'ordinateur maître et s'intégrer dans une boucle de régulation fermée.

Après avoir effectué la mesure de l'EMG, le PDA envoie des informations (totalité de la courbe ou réponse pré-traitée) vers l'ordinateur central qui pilote les pompes d'injection de curare.

Le PDA fonctionne en esclave d'une station de travail (WorkStation). L'ordinateur central interroge périodiquement le système de stimulation-réponse afin de contrôler le degré de bloc neuromusculaire du patient durant l'intervention chirurgicale. La boucle de régulation est de type fermé. L'ordinateur central contrôle également l'injection des pompes à curare.

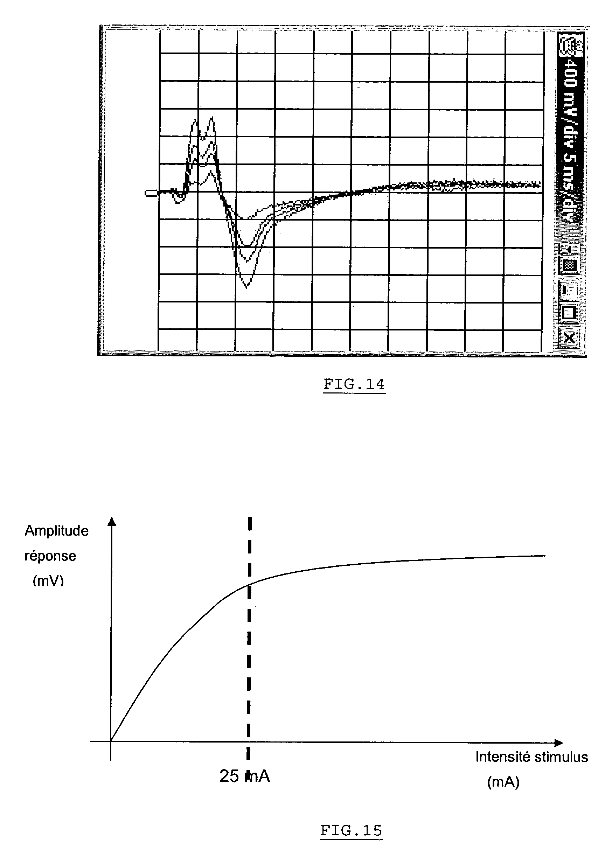

#### 2.2.3 Avantage apporté par l'association stimulateur - système d'acquisition (figure 14)

En électromyographie (EMG), l'évaluation du degré de bloc neuromusculaire se fera en évaluant la réponse du muscle (potentiel évoqué) à une stimulation électrique "supra-maximale" d'un nerf moteur périphérique. Si la réaction d'une fibre musculaire unique est du type "tout ou rien", celle du muscle entier dépend du nombre de fibres actives.

Une stimulation d'intensité suffisante provoquera la réaction de la totalité des fibres musculaire et la réponse obtenue sera maximale en amplitude.

La figure 14 montre l'EMG obtenu lorsqu'on augmente progressivement l'intensité de la source de courant de stimulation.

L'amplitude du signal réponse augmente avec l'intensité des impulsions de courant jusqu'à atteindre la saturation. Cette saturation indique que la totalité des fibres musculaires sont effectivement excitées et la réponse atteint une amplitude maximale.

Vu que l'administration de curare diminue le nombre de fibres actives, on peut mettre en relation l'affaiblissement de la réponse maximale avec l'état de relaxation du muscle.

Pour que cette technique soit efficace, il est indispensable que la totalité des fibres actives soient excitées par la stimulation.

L'intensité de cette stimulation sera donc supérieure de 20 à 25 pour cent de celle pour laquelle on obtient une réponse maximale, d'où le qualificatif "supra-maximale".

La figure 15 montre l'amplitude du signal EMG en fonction de l'intensité des impulsions électriques

L'expérience montre que le seuil, caractérisé par la saturation de l'amplitude, dépend fort d'un muscle à l'autre et même d'un patient à l'autre.

Certains appareils comme le TOF-Watch utilisé actuellement (en accélérométrie) sont programmés par défaut à 50 mA afin de s'assurer d'être au-delà du seuil de saturation et que le muscle soit correctement excité.

Disposant d'une mesure de l'EMG, le microcontrôleur peut déterminer de manière très précise l'intensité des impulsions électriques menant à une excitation supra-maximale.

Un des nombreux avantages à recourir aux EMG pour l'évaluation du taux de relâchement musculaire est la détection beaucoup plus fine du seuil de saturation permettant de diminuer l'intensité des impulsions électriques et, partant, de diminuer ainsi les douleurs post-opératoires.

### 2.3 Point de vue du personnel médical

L'appareil selon l'invention peut être avantageusement utilisé en unité de soins intensifs (USI) ; la diminution de l'encombrement du dispositif (portabilité) permet de réaliser des mesures de potentiels évoqués directement au lit du patient, lors de la période postopératoire en salle de réveil par exemple.

### 2.4 Point de vue utilisateur de tous les jours

L'appareil selon l'invention peut être utile au domicile du patient qui fait de la récupération musculaire.

Afin d'assurer une bonne réhabilitation d'un muscle, les patients peuvent disposer d'un électro-stimulateur portable qu'ils utilisent à domicile (exemple : ceinture pour se muscler).

Il pourrait également s'avérer intéressant de disposer d'un appareil de mesure permettant de contrôler l'évolution du travail de réhabilitation par une mesure d'EMG.

### Revendications

1. Appareil intégré, portable et autonome pour la mesure directe, l'affichage, le traitement et la transmission à distance de signaux électromyographiques (EMG), comprenant :

   - un stimulateur électrique (1) comportant des électrodes pour l'excitation d'un nerf moteur périphérique ;
   - une paire d'électrodes (21, 31), pour l'acquisition de la réponse EMG au niveau du muscle associé à ce nerf périphérique ;
   - une chaîne d'acquisition (2) pilotée par un microcontrôleur (3, 39), présentant des moyens de conditionnement du signal d'entrée, comprenant au moins un préamplificateur différentiel (22, 33), un filtre passe-bande (25, 34) et un convertisseur analogique/numérique (CAN, 26, 310), ladite chaîne d'acquisition (2) étant reliée, via une interface standardisée (5), à un ordinateur (4) comprenant des moyens de mémorisation (9) et d'affichage des signaux EMG acquis ainsi qu'un programme exécutable pour réaliser l'interface avec l'utilisateur (6) et exploiter les données mémorisées, ledit ordinateur (4) étant un PC portable ou un ordinateur de poche de type PDA *(Personal Digital Assistant),* alimenté par batterie et muni de moyens de communication sans fil (7) avec un ordinateur central (8) ;

   **caractérisé en ce que** la chaîne d'acquisition (2) comporte en outre des moyens d'élimination ou d'atténuation d'un artefact de stimulation présent dans le signal EMG, coopérant avec des moyens d'ajustement automatique du gain d'amplification du signal EMG (23, 24, 311, 38), via le microcontrôleur, de manière à ce que le signal EMG couvre la plus grande partie possible de la plage de tension d'entrée du CAN (26, 310), donc avec conservation de la résolution, lorsque l'amplitude du signal EMG diminue.

2. Appareil selon la revendication 1,
   **caractérisé en ce qu'**il est configuré pour effectuer l'acquisition en mode synchronisé sur la stimulation, en mode déclenché par niveau ou en mode continu, le déclenchement étant dans ce dernier cas réalisé à la demande.

3. Appareil selon la revendication 2,
   **caractérisé en ce que**, en mode d'acquisition synchronisé sur la stimulation, la chaîne d'acquisition est pourvue de moyens, de préférence comprenant un relais (32) piloté par le microcontrôleur (39), pour court-circuiter les électrodes d'acquisition (31) pendant la durée de l'apparition de l'artefact de stimulation, tout en synchronisant le début de l'acquisition sur la fin de la stimulation.

4. Appareil selon la revendication 3,
   **caractérisé en ce que** la durée de court-circuitage des électrodes (31) est programmable et comprise entre 1 et 10 000 $\mu$s, de préférence entre 1 et 1000 $\mu$s, et la durée d'acquisition du signal est programmable et comprise entre 1 et 60 000 ms, de préférence entre 1 et 10 000 ms.

5. Appareil selon la revendication 2,
   **caractérisé en ce que**, en vue de l'acquisition en mode déclenché par niveau ou en continu, la chaîne d'acquisition est pourvue d'un deuxième amplificateur (35) pour le traitement du signal après préamplification (33) et filtrage (34), ledit deuxième amplicateur (35) comprenant également des moyens d'ajustement automatique de son gain (312, 38), via le microcontrôleur (39).

6. Appareil selon la revendication 5,
   **caractérisé en ce que** la durée d'acquisition du signal est programmable.

7. Appareil selon l'une quelconque des revendications précédentes, **caractérisé en ce qu'**il est configurable pour permettre l'acquisition et la transmission à distance des données en temps réel.

8. Appareil selon l'une quelconque des revendications précédentes, **caractérisé en ce que** ladite interface standardisée (5) est de type RS-232, RS-485 ou USB.

9. Appareil selon l'une quelconque des revendications précédentes, **caractérisé en ce que** lesdits moyens de mémorisation (9) comprennent une mémoire non-volatile ou une mémoire de masse, de préférence de type SD™ Card ou CompactFlash®.

10. Appareil selon l'une quelconque des revendications précédentes, **caractérisé en ce que** les moyens de communication sans fil (7) comprennent une liaison "Bluetooth", Zigby, "Wifi" ou GSM.

11. Appareil selon l'une quelconque des revendications précédentes, **caractérisé en ce qu'**il est configurable pour travailler en mode "maître/esclave", c'est-à-dire soit de manière complètement autonome -PDA maître- ou esclave de l'ordinateur central pour effectuer des mesures à la demande -PDA esclave.

12. Appareil selon l'une quelconque des revendications précédentes, **caractérisé en ce que** les électrodes (21, 31) sont des électrodes de surface, des électrodes à aiguilles, des implants ou des électrodes actives.

13. Appareil selon l'une quelconque des revendications précédentes, **caractérisé en ce qu'**il comporte des résistances de protection pour limiter le courant de défaut.

14. Appareil selon l'une quelconque des revendications précédentes, **caractérisé en ce qu'**il est configuré pour effectuer automatiquement ou à la demande une mesure d'impédance au niveau des électrodes de stimulation et des électrodes d'acquisition.

15. Appareil selon l'une quelconque des revendications précédentes, **caractérisé en ce que** le programme s'exécutant sur l'ordinateur (4) est entièrement indépendant du système d'exploitation, et est de préférence écrit en Java, une Java Virtual Machine (JVM) adaptée au système d'exploitation étant fournie pour ce faire.

16. Appareil selon l'une quelconque des revendications 1 à 15, **caractérisé en ce que** le stimulateur (1) est configuré pour travailler en mode programmé, une série de séquences d'impulsions rectangulaires reparamétrables étant préenregistrées en mémoire, de préférence de type ST (Single Twich), TOF (Train of Four), TS (Tetanic Stimulation) ou DBS (Double Burst Stimulation).

**17.** Appareil selon la revendication 16,
**caractérisé en ce que** l'amplitude des impulsions est comprise entre 0 et 150 mA dans 5kΩ, leur largeur entre 0 et 1000 μs et la période séparant deux séquences successives entre 10 et 60 secondes.

**18.** Appareil selon l'une quelconque des revendications 1 à 15, **caractérisé en ce que** le stimulateur (1) est configuré pour travailler en mode programmable, l'utilisateur pouvant choisir la forme, la fréquence et l'amplitude des impulsions.

**19.** Appareil selon la revendication 18,
**caractérisé en ce que** les impulsions sont sinusoïdales, triangulaires, rectangulaires et/ou arbitraires.

**20.** Appareil selon la revendication 18 ou 19,
**caractérisé en ce que** le stimulateur (1) peut être configuré pour travailler en mode mono-impulsion, multi-impulsions ou continu.

**21.** Appareil selon l'une quelconque des revendications précédentes, **caractérisé en ce que** le programme exécutable sur l'ordinateur (4) permet directement un affichage et une analyse, en temps réel et/ou en post-traitement, d'au moins un signal EMG acquis ou d'au moins un paramètre lié audit signal, comprenant par exemple une détermination de l'amplitude crête-à-crête du signal ou de la surface du signal redressé, y compris le rapport de telles grandeurs avec des valeurs de référence, ainsi qu'une analyse spectrale.

**22.** Procédé pour éliminer un artefact de stimulation présent dans un signal EMG d'entrée et pour ajuster automatiquement le gain d'amplification dudit signal avec conservation de la résolution du convertisseur analogique/numérique dans l'appareil de mesure, selon la revendication 1, lorsque cet appareil est utilisé en mode de déclenchement synchronisé sur la stimulation,
**caractérisé par** la séquence automatisée d'étapes successives suivantes :

- on court-circuite les électrodes d'acquisition pendant une durée supérieure à la durée d'apparition de l'artefact de stimulation ;
- on procède à l'acquisition en synchronisant le début de l'acquisition sur la fin du signal de stimulation ;
- on préamplifie le signal obtenu avec un gain $G_{MAX}$ tel que :

$$G_{MAX} = \frac{V_{REF}}{V_{EMG}} ,$$

où $V_{EMG}$ est l'amplitude crête de l'EMG et $V_{REF}$ est la demi-plage d'entrée du CAN, en valeur absolue ;

- on filtre le signal amplifié.

**23.** Procédé pour atténuer un artefact de stimulation présent dans un signal EMG d'entrée et pour ajuster automatiquement le gain d'amplification dudit signal avec conservation de la résolution du convertisseur analogique/numérique dans l'appareil de mesure, selon la revendication 1, lorsque cet appareil est utilisé en mode de déclenchement par niveau, **caractérisé par** la séquence automatisée d'étapes successives suivantes :

- on procède à l'acquisition du signal ;
- on préamplifie le signal acquis avec un gain respectant la condition :

$$G_{MAX,1} = \frac{V_{REF}}{V_{ARTEFACT}} ,$$

où $V_{ARTEFACT}$ est l'amplitude crête de l'artefact et $V_{REF}$ est la demi-plage d'entrée du CAN, en valeur absolue, ce qui donne un signal de sortie EMG amplifié

$$V_0 = G_{MAX,1} \cdot V_{EMG} \quad ;$$

- on filtre ce signal avec un filtre passe-bande choisi pour ne conserver qu'une bande d'énergie utile du signal EMG, de manière telle que l'amplitude de l'artefact de stimulation devienne inférieure à celle du signal EMG ;
- on amplifie le signal filtré avec un gain respectant la condition :

$$G_{MAX,2} = \frac{V_{REF}}{V_0} \quad .$$

FIG.1

FIG.2A

motif

Ta = temps d'attente avant de délivrer le prochain motif (0 –

mono-

multi-

X nombre Sequence

continu

FIG.2B

FIG.3

FIG.4

FIG.5

FIG.6

FIG.7

FIG.8

EP 1 656 883 A1

FIG.9

26

Bit de commande
du µC

FIG.10A

Δ

δ

t₀   t₁   t₂        t₃                    t₄    t

FIG.10B

## Mode 1 (ou synchro)

$V_{IN}$ (mV)

$V_{ARTEFACT}$

5

$V_{EMG}$

t

⇓ Court-circuitage des électrodes (élimination artefact)

$V_{IN}$ (mV)

$V_{ARTEFACT}$

5

$V_{EMG}$

t

⇓ Pré-amplification (optimale)

$V_0$ (V)

$$G = \frac{V_{ref}}{V_{EMG}}$$

Vref +

Vref −

t

⇓ Filtrage (élimination du bruit)

$V_A$ (V)

Vref +

Vref −

t

## Mode 2 (par niveau)

$V_{IN}$ (mV)

$V_{ARTEFACT}$

5

$V_{EMG}$

t

⇓ Pré-amplification (gain limité)

$V_0$ (V)

$$G = \frac{V_{ref}}{V_{ARTEFACT}}$$

Vref +

Vref −

t

⇓ Filtrage (atténuation artefact)

$V_F$ (V)

Vref +

$V_0$

Vref −

t

⇓ Amplification (optimale)

$V_A$ (V)

$$G = \frac{V_{ref}}{V_{EMG}}$$

Vref +

Vref −

t

FIG.10C

28

Niveau de déclenchement
Niveau de référence

FIG.10D

$\Delta$

$t_0 - \epsilon$    $t_0$    $t_1$    $t$

FIG.10E

$$\Delta$$

$t_0 \qquad t_1 \qquad t$

FIG.10F

EMG 1    EMG 2

EMG 3

1    2    3

FIG.11

FIG.12

FIG.13

EP 1 656 883 A1

FIG.14

Amplitude réponse (mV)

25 mA

Intensité stimulus (mA)

FIG.15

**Office européen**

**des brevets**

## RAPPORT DE RECHERCHE EUROPEENNE

Numéro de la demande

EP 04 44 7248

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | US 6 083 156 A (LISIECKI ET AL) 4 juillet 2000 (2000-07-04) * colonne 4, ligne 5-50 * * colonne 6, ligne 62 - colonne 7, ligne 5 * * colonne 7, ligne 23-39 * * colonne 8, ligne 33-40 * * figure 2 * ----- | 1,2,5-21 | A61B5/0488 A61B5/04 |
| Y | MILLARD R E ET AL: "A gated differential amplifier for recording physiological responses to electrical stimulation" JOURNAL OF NEUROSCIENCE METHODS NETHERLANDS, vol. 44, no. 1, 1992, pages 81-84, XP002322320 ISSN: 0165-0270 page 83, methods ----- | 1,2,5-21 | |
| A | US 5 010 888 A (JADVAR ET AL) 30 avril 1991 (1991-04-30) * colonne 7, ligne 54 - colonne 8, ligne 3 * ----- | 3,4,22 | |
| Y | US 5 300 096 A (HALL ET AL) 5 avril 1994 (1994-04-05) | 16-20 | DOMAINES TECHNIQUES RECHERCHES (Int.Cl.7) A61B G06F |
| A | * colonne 1, ligne 62 - colonne 2, ligne 30 * * colonne 3, ligne 31-68; figures 8-10 * ----- | 1-15, 21-23 | A61N H03G H03F |
| A | US 6 195 585 B1 (KARUNASIRI RANKIRI TISSA ET AL) 27 février 2001 (2001-02-27) * colonne 1, ligne 34-51 * * colonne 2, ligne 11-16 * * colonne 2, ligne 50-57 * * colonne 3, ligne 44-63 * * colonne 13, ligne 61 - colonne 14, ligne 41 * ----- | 1-23 | |

Le présent rapport a été établi pour toutes les revendications

| Lieu de la recherche | Date d'achèvement de la recherche | Examinateur |
|---|---|---|
| La Haye | 24 mars 2005 | Lommel, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**

EP 04 44 7248

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-03-2005

| Document brevet cité au rapport de recherche | | Date de publication | Membre(s) de la famille de brevet(s) | | Date de publication |
|---|---|---|---|---|---|
| US 6083156 | A | 04-07-2000 | AUCUN | | |
| US 5010888 | A | 30-04-1991 | AU<br>WO | 3297089 A<br>8909514 A1 | 16-10-1989<br>05-10-1989 |
| US 5300096 | A | 05-04-1994 | AUCUN | | |
| US 6195585 | B1 | 27-02-2001 | AUCUN | | |

EPO FORM P0460

Pour tout renseignement concernant cette annexe : voir Journal Officiel de l'Office européen des brevets, No.12/82